# EUROPEAN PATENT SPECIFICATION

(11) **EP 1 893 254 B1**
(45) Date of publication and mention of the grant of the patent: **04.10.2017**
(21) Application number: 06754935.2
(22) Date of filing: 28.04.2006
(51) Int. Cl.: A61M 1/06

(54) **HAND HELD BREAST PUMP**
IN DER HAND GEHALTENE BRUSTPUMPE
TIRE-LAIT MANUEL

(30) Priority: 29.04.2005 GB 0508904
(43) Date of publication of application: 05.03.2008
(73) Proprietor: Avent Limited, London N11 1SS (GB)
(72) Inventor: WORTLEY, Mark, Suffolk, CO10 2PU (GB); WILLIAMS, Roger, Hertfordshire, EN10 7HB (GB); ATKINS, Edward, London, NW3 7RG (GB)
(74) Representative: Damen, Daniel Martijn
(86) International application number: PCT/EP2006/061946
(87) International publication number: WO 2006/117352

(56) References cited:
- EP-A- 0 733 376
- EP-A- 1 502 610
- DE-A1- 10 132 516
- US-A- 5 947 923
- US-A1- 2004 087 898
- US-A1- 2005 068 459
- US-B1- 6 673 036

## Description

The present invention relates to a hand held powered breast pump and a hand held breast pump system.

WO-A-01/47577 discloses a programmable powered breast pump apparatus. The disclosed breast pump apparatus is programmed by recording control data on or in a data carrier which is then inserted into the breast pump apparatus. The control data is read from the data carrier and controls the pumping cycle.

The breast pump apparatus disclosed in WO-A-01/47577 suffers from the disadvantage that the programming process is complex.

US-A-5571084 discloses a powered breast pump in which the user has some limited control over the pumping cycle. The limited control that the user has is itself a problem.

EP-A-1502610 discloses a powered breast pump which has learn and run mode of operation.

It is known from EP0733376 to provide a hand held, powered breast pump comprising:
a body having a breast-receiving portion and means for attaching a vessel to receive expressed milk;
piston means, including an actuating element, for effecting pumping; and
a motor unit mountable to the body and including a motor and a drive mechanism for reciprocally driving the piston means.

According to the present invention, there is provided a hand held, powered breast pump comprising:
a body having a breast-receiving portion and means for attaching a vessel to receive expressed milk;
piston means, including an actuating element, for effecting pumping; and a motor unit including a motor and a driving mechanism for reciprocally driving the piston means, wherein the drive mechanism is configured to connect to said actuating element when brought into contact therewith, and wherein the drive mechanism is a crank mechanism and includes connecting rod means and said connection is effected by relative resilient deflection and
recovery between the connecting rod means and said actuating element.. Preferably, the actuating element is a resiliently deflectable projection projecting from the piston means. However, the resilience may be provided in the connecting rod.

Preferably, the projection has a bulge and the connecting rod means includes means for receiving said bulge so as to couple the connecting rod means and the projection operatively. However, the bulge could be provided on the connecting rod with the means for receiving the bulge being provided on the projection. More preferably, the connecting rod means has a coupling portion shaped to surround the projection partially and the means for receiving the bulge comprises a recess or slot in said coupling portion. Other coupling mechanisms, for instance sprung cleft and magnetic couplings, could be used instead.

Preferably, the piston means comprises a resilient diaphragm but may be a rigid piston. In the case of a diaphragm-type piston means, it is preferable that:
the drive mechanism is a crank mechanism and includes connecting rod means;
the actuating element projects from the diaphragm; and
the connecting rod means is configured such that said connection to said actuating element is effected by the actuating element first being deflected and then recovering, due to the resilience of the diaphragm.

Preferably, a control circuit, configured to effect pumping by alternating the direction of the motor, is included.

The body may include a pumping chamber in which the piston means operates. However, the pumping chamber may be in the motor unit.

The hand held breast pump may be battery powered and, if so, may include a boost regulator for regulating the voltage supplied to the motor.

An embodiment of the present invention will now be described, by way of example, with reference to the accompanying drawings, in which:
Figure 1 shows a breast pump system according to the present invention;
Figure 2 is a sectional side view of the powered head in Figure 1 mounted to a body;
Figure 3 is a top view of the powered head and part of a body with the head's cover removed;
Figure 4 is a block diagram of the battery pack in Figure 1;
Figure 5 is a block diagram of the powered head and its control system;
Figures 6(a) to 6(c) illustrate the process of connecting the connecting rod of the powered head shown in Figure 2 to its diaphragm;
Figure 7 is a flowchart illustrating the operation of the system in Figure 1;

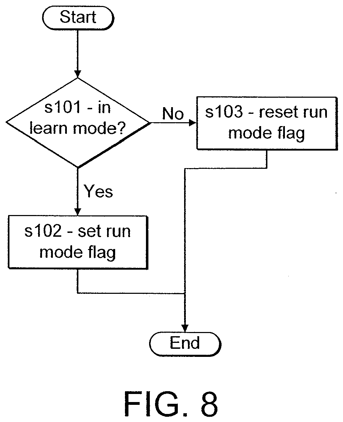

Figure 8 is a flowchart illustrating mode changing of the system in Figure 1;
Figure 9 is a plot illustrating two exemplary pumping cycles during "learn" mode operation of the system in Figure 1;
Figure 10 is a flowchart illustrating part of the "learn" mode operation in detail;
Figure 11 is a plot illustrating three "run" mode pumping cycles of the system of Figure 1;
Figure 12 is a sectional side view of a second powered head according to the present invention; and
Figure 13 is a front view of the second powered head shown in Figure 12.

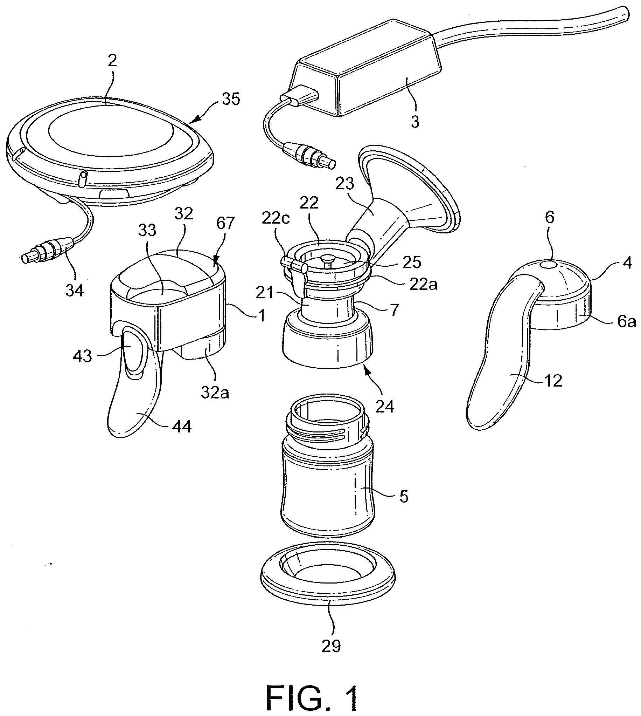

Referring to Figure 1, a breast pump system according to the present invention comprises a powered head 1, a battery pack 2, a mains PSU 3, a manual head 4, a feeding bottle 5, a base 29 for the feeding bottle 5 and a body 7.

The manual head 4, the body 7 and the feeding bottle 5 are very similar to the components of the Avent (RTM) ISIS (RTM) breast pump product.

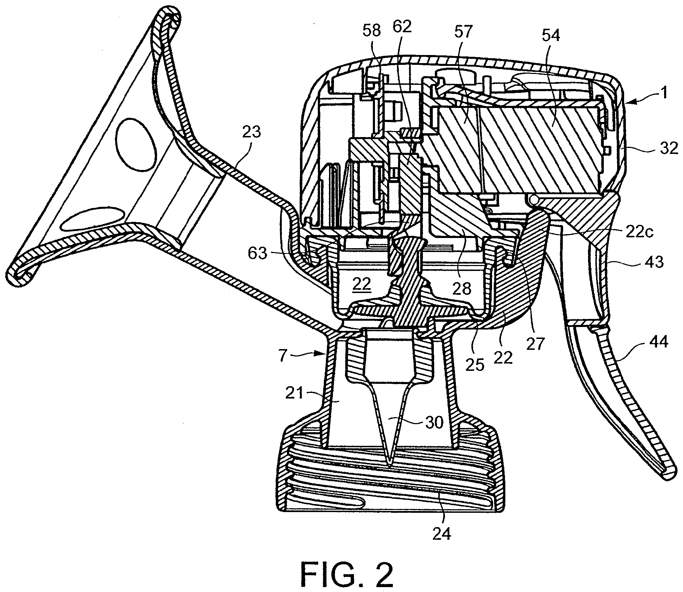

The body 7 comprises a neck 21, a cup 22 supported on the neck 21 and a trumpet 23 projecting from the side of the cup 22. The bottom of the neck is flared and provided with an internal thread 24 (see Figure 2) for screwing the body 7 onto a bottle 5. The trumpet 23 is inclined upwards and, in use, receives the nipple and surrounding breast portion of the user and is sealed thereby. A passageway extends from the open, distal end of the trumpet 23 through the bottom of cup 22 and the neck 21 to the open, bottom end of the neck 21. A duckbill valve 30 (see Figure 2) is provided between the cup 22 and the neck 21. The valve 30 is closed when the pressure in the cup 22 drops relative to the pressure in the neck 21 and opens when the pressure in the cup 22 rises to exceed the pressure in the neck 21.

The manual head 4 comprises a simple domed cover 6 and a lever 12. The domed cover 6 has skirts 6a on opposite sides that clip over respective radially extending flanges 22a on the cup 22 of the body 7. The lever 12 is for operating a diaphragm 25 mounted in the cup 22 of the body 7 and is configured to pivot on a fulcrum provided by a projection 22c rising from the back edge of the cup 22 of the body 7.

Further information regarding the operation of the manual head 4 in combination with the body 7 and a feeding bottle 5 can be obtained from EP-A-0733376.

Referring to Figure 2, the diaphragm 25, received within the cup 22, is cup-shaped and formed from elastomeric material. The rim of the diaphragm 25 is turned back on itself to form a channel 27 which receives the rim of the cup 22 to secure the diaphragm in the cup 22.

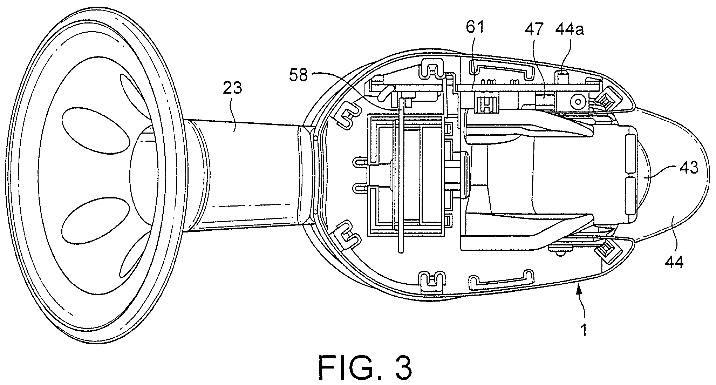

Referring also to Figure 3, the powered head 1 comprises a base 28, which is large enough to cover the interior of the diaphragm 25 and has an annular flange that fits tightly into the top of the diaphragm, a cover 32 is clipped over the base 28. The cover 32 has two skirts 32a (See Figure 1) that clip over the radially extending flanges 22a on the cup 22.

An arm 43 and a lever 44 extend from the back of the housing 26, through a cut away in the cover 32. The overall form of the arm 43 and the lever 44 resembles that of the operating lever 12 of the manual head 4. The lever 44 has a longitudinal slot in its proximal end and the arm 43 extends along the slot 45, substantially filling it. The proximal end of the lever 44 is pivoted within the cover 32.

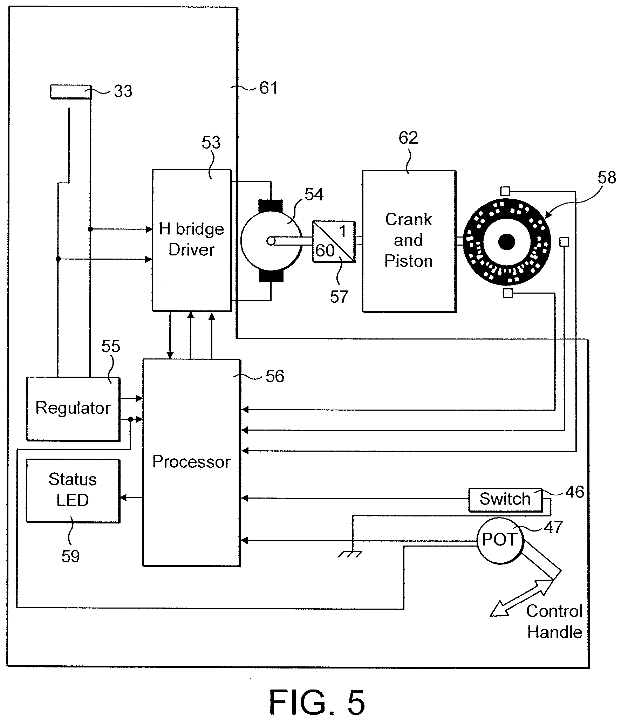

The arm 43 is pivotably mounted within the cover 32 so that pressing the arm 43 towards the cup 22 closes a switch 46 (see Figure 5).

The lever 44 is biased outwards by a spring (not shown) within the cover 32.

Referring back to Figure 1, the battery pack 2 can be carried in a pouch (not shown). The powered head 1 has a socket 33 which opens through the cover 32 and the battery pack 2 has a plug 34 which is received in the socket 33 to supply power to the powered head 1.

The mains PSU 3 is conventional and can be plugged directly into the socket 33 in the powered head 1 as an alternative to battery power. Also, the mains PSU 3 can be plugged into a socket 35 in the battery pack 2 for recharging batteries therein and power from the mains PSU 3 may be conveyed through the battery pack 2 to the powered head 1.

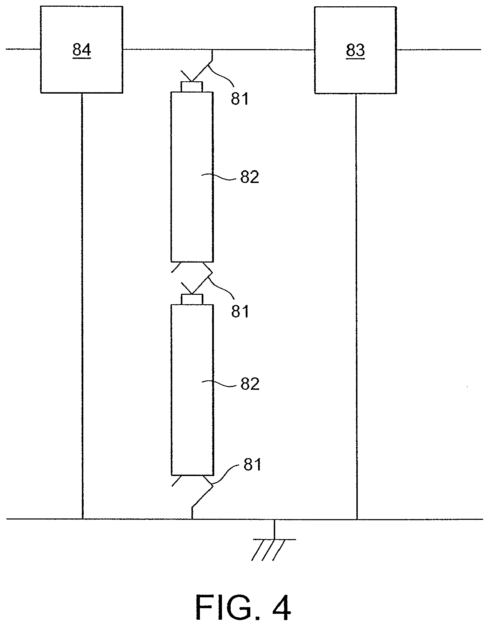

Referring to Figure 4, the battery pack 2 includes terminals 81 for contacting batteries 82 (two shown but there may be more), a boost regulator 83 and a charging circuit 84. The use of the boost regulator 83 results in more consistent pump operation as the batteries 82 discharge.

Referring to back to Figures 2 and 3 and to Figure 5, the powered head 1 houses a printed circuit board 61, which carries the unit's control circuit, a motor 54, a crank mechanism 62, a reduction gear 57 and a rotary encoder 58.

The power supplied through the socket 33 is provided to an H-bridge circuit 53, which provides power to the motor 54, and to a regulator 55. The regulated output of the regulator 55 powers the processing circuit 56, which is built around a microcontroller.

The motor 54 drives the crank mechanism 62 back and forth over a range of up to 180° via the reduction gear 57.

The crank mechanism 62 includes a connecting rod 63 which connects to a projection 64 from the diaphragm 25. The head of the projection 64 is formed into a flattened bulb.

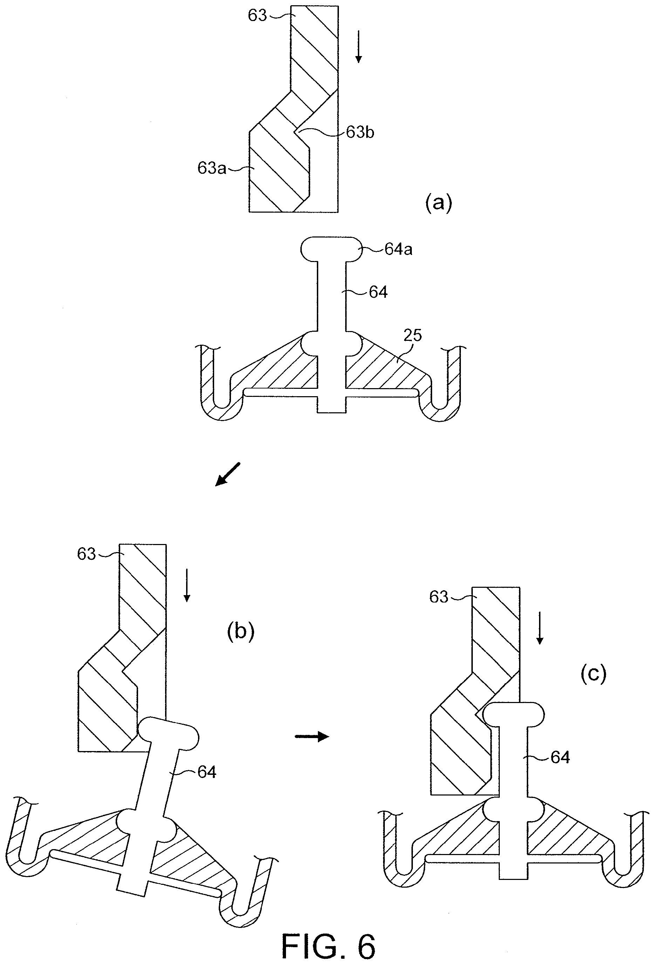

Referring to Figures 6(a) to 6(c), the lower end 63a of connecting rod 63 has a semi-circular cross-section. A transverse slot 63b is formed a short distance from the bottom of the connecting rod 63 for receiving the bulb 64a at the top of the projection 64.

The powered head 1 is mounted to the cup 22 by aligning it with the cup 22 and pressing it into engagement. During this process, if the connecting rod 63 is at bottom dead centre, the bottom of the connecting rod 63 deflects the projection 64 (Figure 6(b)) and when the bulb 64a of the projection 64 is aligned with the slot 63b in the connecting rod 63, the resilience of the diaphragm 25 moves the bulb 64a of the projection 64 into the slot 63b, returning the projection 64 to its upright position and drivingly coupling the connecting rod 63 and the projection 63 (Figure 6(c)).

If the connecting rod 63 is not at bottom dead centre when the powered head 1 is mounted to the cup 1, the connecting rod 63 engages with the projection 64 as the connecting rod passes through bottom dead centre for the first time after the head 1 is mounted to the cup 22. In this case, as the connecting rod 63 passes through bottom dead centre, the bottom of the connecting rod 63 deflects the projection 64 (Figure 6(b)) and when the bulb 64a of the projection 64 is aligned with the slot 63b in the connecting rod 63, the resilience of the diaphragm 25 moves the bulb 64a of the projection 64 into the slot 63b, returning the projection 64 to its upright position and drivingly coupling the connecting rod 63 and the projection 63 (Figure 6(c)).

The resilience of the diaphragm 25 also allows the housing 26 to be pulled off the cup 22.

In contrast, the manual head 4 is mounted by engaging the projection 64 with a fork at one end of the operating lever 12 so that the bulb 64a of the projection 64 is above the fork, seating the lever 12 on its fulcrum and clipping on the cover 6. Thus, when the mounted operating lever 12 is operated by a user, the projection 64 is raised, taking with it the main part of the diaphragm 25. When the user releases the operating lever 12, the diaphragm 25 returns to its rest position.

Referring again to Figures 2, 3 and 5, when the powered head 1 is mounted on the cup 22, running of the motor 54 causes the crank mechanism 62 to raise and lower the diaphragm 25 and effect pumping. The position of the crank mechanism 62 is sensed by the rotary encoder 58.

A status LED 59 is controlled by an output from the control circuit 56 to provide feedback to the user. The light from the LED 59 is conducted from the printed circuit board 61 and to a lens 67 (see Figure 1) by an optical waveguide (not shown) so that it can be seen by a user.

The switch 46 is coupled between an interrupt input of the processing circuit 56 and ground. The pivot arrangement of the lever 44 includes a stub 44a that passes through a preset-type potentiometer 47, mounted on the printed circuit board 61, so that the wiper of the potentiometer 47 moves when the lever 44 is pivoted. The potentiometer 47 is coupled between the positive voltage output of the regulator 55 and ground, and the wiper of the potentiometer 47 is connected to an analogue-to-digital converter input of the processing circuit 56.

The processing circuit 56 provides motor speed and direction signals to the H-bridge circuit 53 to control the movement of the motor 54.

The operation of the system will now be described.

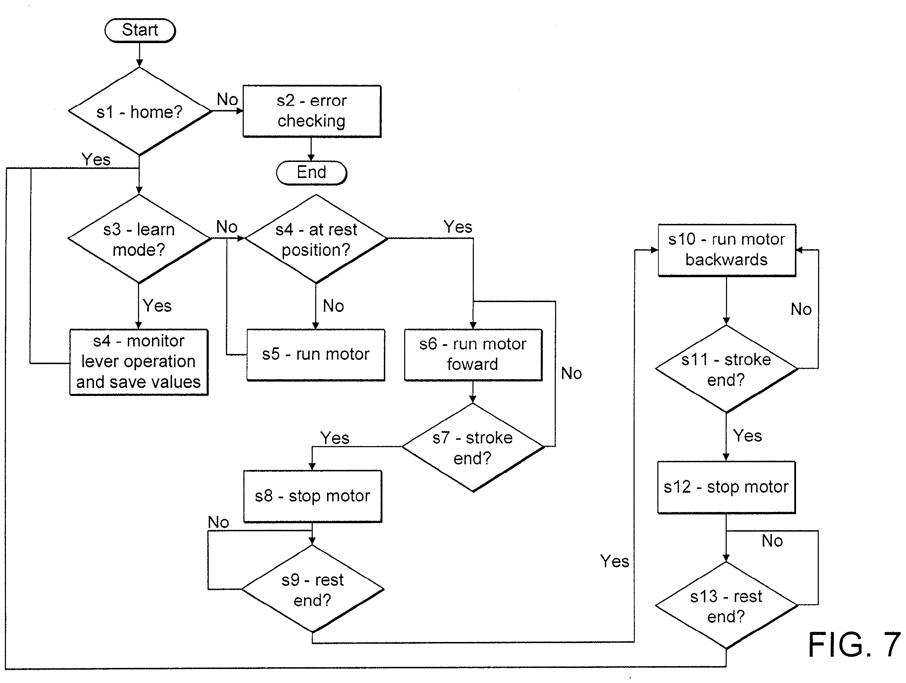

Referring to Figure 7, when the system is turned on, the processing circuit 56 determines whether the crank is in its home position by comparing the output of the rotary encoder 58 with a home reference value, i.e. 0 (step s1). If the crank mechanism 62 is not in its home position, the processing circuit performs an error checking routine and signals any errors using the status LED 59 (step s2) and halts the system.

The system enters "learn" mode by default. However, referring to Figure 7, closing the switch 46 while the system is in "learn" mode switches it to "run" mode by setting a run mode flag (steps s101 and s102) and closing the switch 46 while the system is in "run" mode will switch it back to "learn" mode by resetting the run mode flag (steps s101 and s103).

In "learn" mode (step s3), the motor 54 is controlled by the processing circuit 56 in a simple servo loop (step s4). As the user presses the lever 44 towards the body 7, the wiper of the potentiometer 47 moves changing the output voltage of the potentiometer 47. This voltage is digitised and compared with the output of the rotary encoder and any error is used to generate control signal to drive the motor 56 so as to remove the error. Pressing the lever 44 causes the motor 54 to move the diaphragm 25 up and apply a sucking force in the breast receiving trumpet 23, and allowing the lever 44 to move towards its rest position causes the motor 54 to move the diaphragm 25 down, relieving the suction. Thus, the pressure in the trumpet 23 is set by the position of the lever 44 and the user can experiment by pressing and releasing the lever 44 until a comfortable and effective cycle has been discovered.

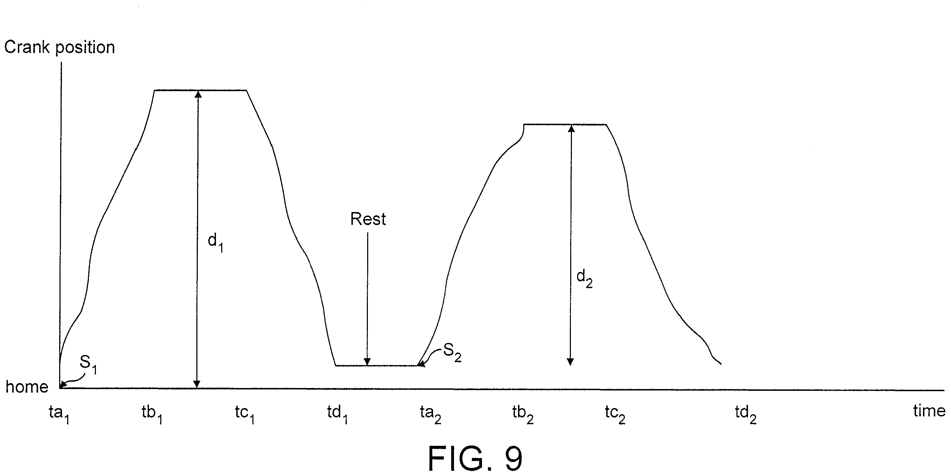

Referring to Figure 9, during operation in the "learn" mode, the processing circuit 56 monitors the times of the start of each suctions stroke (ta), the end of each suction stroke (tb), the start of each relaxation stroke (tc) and the end of each relaxation stroke (td). The processing circuit 56 also monitors the start position (s) and lengths (d) of each suction stroke.

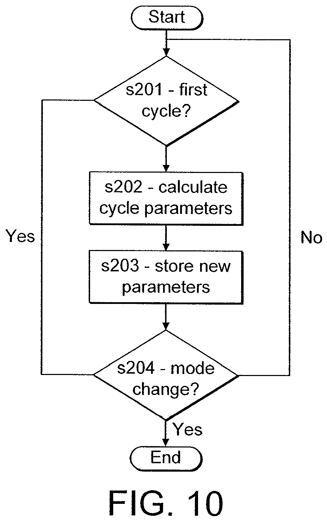

Referring to Figure 10, at the end of the second and each subsequent relaxation stroke, i.e. at tdₙ, (step s201) the processing circuit 56 calculates the suction stroke velocity (i.e. d/(tb-ta)), the suction hold period, the relaxation stroke velocity (i.e. d/td-tc)) and the rest period of the previous cycle (if any) are calculated from the monitored times for the current and preceding cycles (step s202) and stored (step s203). The state of the run mode flag is then checked (step s204) and if the mode has changed to "run" mode, "learn" mode is left otherwise the process returns to step s101.

When the system is switched to "run" mode by the user closing the switch 46, the processing circuit 56 controls the motor 54 in accordance with the stored velocities and periods and the stored stroke and depth value.

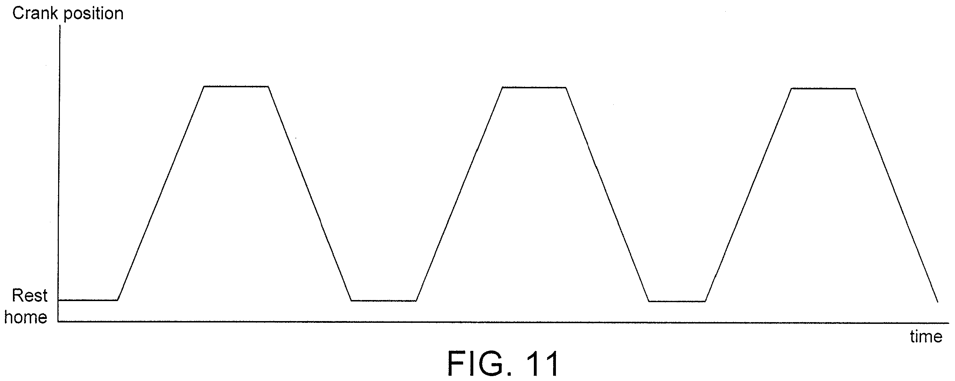

Referring to Figures 7 and 11, if the system is determined to be in "run" mode (step s3), the processing circuit 56 determines whether the crankshaft is in the rest position, i.e. the suction stroke start position, (step s4) and, if it is not, outputs a stream of pulses to the H-bridge circuit 53, for setting the speed of the motor 54, and a direction signal so that the diaphragm 25 moves at the stored average suction velocity (step s5) until the rest position is reached. When the crankshaft is in the rest position (step s4), the processing circuit 56 begins to output a stream of motor speed control pulses to the H-bridge circuit 53 together with a forward signal for causing the motor 54 to run in a first direction (step s6). These pulses and the forward signal are output until the output of the rotary encoder 58 matches the sum of the rest position and the stored stroke depth value (step s7) at which time the motor is stopped (step s8). When the motor has been stopped, the processing circuit 56 does not output anymore speed control pulses until a period corresponding to the stored suction hold period has expired (step s9). When the suction hold period has expired (step s9), the processing circuit 56 outputs speed control pulses, corresponding to the stored average relaxation stroke velocity, to the H-bridge circuit 53 together with a reverse signal (step s9). These signals cause the motor to be driven in a second, opposite direction, allowing the diaphragm 25 to relax, and are terminated when the rotary encoder 58 indicates that the diaphragm 25 has returned to its rest position (steps s11 and s12). The processing circuit 56 does not then output any further motor control signals and when the stored average rest period has expired (step s13), it returns to step s3.

The system can be operated by a user in "learn" mode without switching to "run" mode. Thus, the system also provides a power-assisted breast pump which gives the user continuous control over the pumping cycle as with manual breast pumps but without the physical effort required by a manual breast pump.

It will be appreciated that the present invention can be implemented in other ways. For example, the "run" mode cycles could follow the "learn" mode cycle with greater fidelity. This could be achieved by recording the position of the crankshaft at a plurality of instants during each stroke in "learn" mode. The "run" mode cycle could be established by taking averages of cycle parameters obtained from a plurality of learn mode cycles. For convenience, the user input means mimics the actuator of a manual breast pump. This arrangement could be replaced by a foot switch and pedal rather like that of a sewing machine or a separate handheld control unit. The user inputs may be communicated to the processing circuit wirelessly, e.g. using IR.

An alternative to the suction start parameter and stroke length parameter combination would be a rest position parameter and suction hold position parameter.

A second embodiment is electrically the same as the first embodiment described above but is physically configured for use on the body 107 of a Medela (RTM) Harmony manual breast pump.

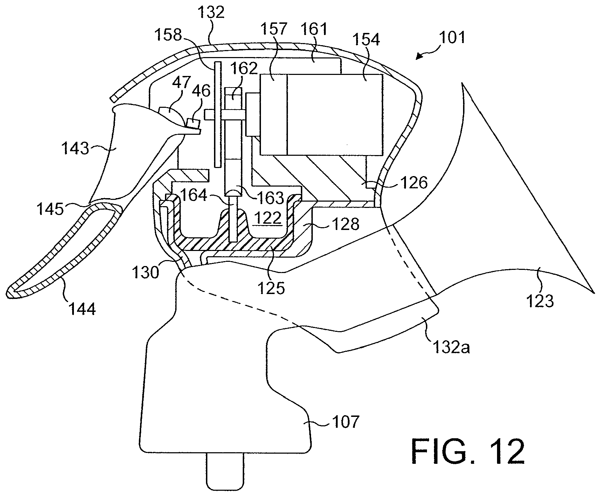

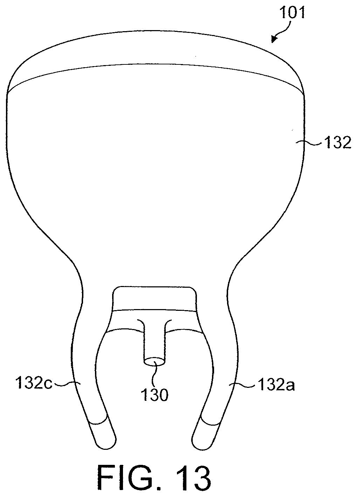

Referring to Figures 12 and 13, a second powered head 101 comprises a base 128, which includes a pumping chamber 122 containing a diaphragm 125, a chassis 126 mounted to the base 128 and a cover 132 that encompasses the base 128 and the chassis 126. The cover 132 has a pair of cheek pieces 132a that clip around the root of the trumpet 123 of the breast pump body 107 to hold the powered head 101 in place. A tube 130 projects from the bottom of the base 128 for insertion into a corresponding hole in the breast pump body 107. The upper end of the tube 130 opens into the pumping chamber 122.

An arm 143 and a lever 144 extend from the back of the housing 126, through a cut away in the cover 132. The overall form of the arm 143 and the lever 144 resembles the operating lever 12 of the manual head 4, described above. The lever 144 has a longitudinal slot 145 in its proximal end and the arm 143 extends along the slot 145, substantially filling it. The proximal end of the lever 144 is pivoted within the cover 132.

The arm 143 is pivotably mounted within the cover 132 so that pressing the arm 143 towards the breast pump body 107 closes a switch 46 (see Figure 5).

The lever 144 is biased outwards by a spring (not shown) within the cover 132 and is drivingly coupled to the wiper of the potentiometer 47 (see also Figure 5).

The powered head 101 houses a printed circuit board 161, which carries the unit's control circuit, a motor 154, a crank mechanism 162, a reduction gear 157 and a rotary encoder 158.

The motor 154 drives the crank mechanism 162 back and forth over a range of up to 180° via the reduction gear 157.

The crank mechanism 162 includes a connecting rod 163 which connects to a projection 164 from the diaphragm 125.

The operation of the second powered head 101 for expressing milk is the same as the operation of the first powered head 1 described above.

It will be appreciated that many modifications may be made to the embodiments described above. For example, the diaphragm could be replaced by a rigid piston or be inverted with the motor driving the periphery of the diaphragm up and down. As mentioned above, a lead screw mechanism can be used to drive the piston or diaphragm. The need for a crank mechanism, a screw mechanism or any other rotary to linear motion conversion mechanism can be avoided by use of linear motor instead of the rotary motor described above.

## Claims

1. A hand held, powered breast pump comprising:
a body (7) having a breast-receiving portion (23) and means for attaching a vessel to receive expressed milk;
piston means (25), including an actuating element (64), for effecting pumping; and
a motor unit (1) including a motor (54) and a drive mechanism for reciprocally driving the piston means (25), wherein the drive mechanism is configured to connect to said actuating element when brought into contact therewith, **characterised in that** the drive mechanism is a crank mechanism (62) and includes connecting rod means (63) and said connection to said actuating element (64) is effected by relative resilient deflection and recovery between the connecting rod means (63) and said actuating element (64).

2. A hand held, powered breast pump according to claim 1, wherein the actuating element is a resiliently deflectable projection (64) projecting from the piston means (25).

3. A hand held, powered breast pump according to claim 2, wherein the projection (64) has a bulge (64a) and the connecting rod means (63) includes means for receiving said bulge (64a) so as to couple the connecting rod means (63) and the projection (64) operatively.

4. A hand held, powered breast pump according to claim 3, wherein the connecting rod means (63) has a coupling portion (63a) shaped to surround the projection (64) partially and the means for receiving the bulge (64a) comprises a recess or slot (63b) in said coupling portion (63a).

5. A hand held, powered breast pump according to any preceding claim, wherein the piston means comprises a resilient diaphragm (25).

6. A hand held, powered breast pump according to claim 5, wherein:
the drive mechanism is a crank mechanism (62) and includes connecting rod means (63);
the actuating element (64) projects from the diaphragm (25); and
the connecting rod means (63) is configured such that said connection to said actuating element (64) is effected by the actuating element (64) first being deflected and then recovering, due to the resilience of the diaphragm (25).

7. A hand held, powered breast pump according to any preceding claim, including a control circuit (61) configured to effect pumping by alternating the direction of the motor (54).

8. A hand held, powered breast pump according to any preceding claim, wherein the body (7) includes a pumping chamber (122) in which the piston means (25) operates.

9. A battery-powered hand held breast pump according to any preceding claim.

10. A battery-powered hand held breast pump according to claim 9, including a boost regulator (83) for regulating the voltage supplied to the motor (54).

## Patentansprüche

1. Handgehaltene strombetriebene Brustpumpe, umfassend:
ein Gehäuse (7), das einen Brustaufnahmeabschnitt (23) und Mittel zum Befestigen eines Gefäßes zum Aufnehmen von abgepumpter Milch umfasst;
Kolbenmittel (25), einschließlich eines Betätigungselements (64), zum Bewirken einer Pumpentätigkeit; und
eine Motoreinheit (1), einschließlich eines Motors (54) und eines Antriebsmechanismus zum wechselseitigen Antreiben der Kolbenmittel (25),
wobei der Antriebsmechanismus dazu eingerichtet ist, sich mit dem Betätigungselement zu verbinden, wenn er mit demselben in Berührung gebracht wird,
**dadurch gekennzeichnet, dass** der Antriebsmechanismus ein Kurbelmechanismus (62) ist und Verbindungsstangenmittel (63) einschließt, und die Verbindung mit dem Betätigungselement (64) durch relative elastische Verformung und Rückverformung zwischen dem Verbindungsstangenmittel (63) und dem Betätigungselement (64) bewirkt wird.

2. Handgehaltene strombetriebene Brustpumpe nach Anspruch 1, wobei das Betätigungselement ein elastisch verformbarer Vorsprung (64) ist, der von den Kolbenmitteln (25) vorspringt.

3. Handgehaltene strombetriebene Brustpumpe nach Anspruch 2, wobei der Vorsprung (64) einen Wulst (64a) besitzt und das Verbindungsstangenmittel (63) Mittel zum Aufnehmen des Wulstes (64a) besitzt, um das Verbindungsstangenmittel (63) und den Vorsprung (64) betriebsmäßig zu koppeln.

4. Handgehaltene strombetriebene Brustpumpe nach Anspruch 3, wobei das Verbindungsstangenmittel (63) einen Kopplungsabschnitt (63a) besitzt, der dazu ausgeformt ist, den Vorsprung (64) teilweise zu umgeben, und das Mittel zum Aufnehmen des Wulstes (64a) eine Vertiefung oder einen Schlitz (63b) in dem Kopplungsabschnitt (63a) umfasst.

5. Handgehaltene strombetriebene Brustpumpe nach einem vorhergehenden Anspruch, wobei das Kolbenmittel eine elastische Membran (25) umfasst.

6. Handgehaltene strombetriebene Brustpumpe nach Anspruch 5, wobei:
der Antriebsmechanismus ein Kurbelmechanismus (62) ist und Verbindungsstangenmittel (63) einschließt;
das Betätigungselement (64) von der Membran (25) vorspringt; und
das Verbindungsstangenmittel (63) derart eingerichtet ist, dass die Verbindung mit dem Betätigungselement (64) dadurch bewirkt wird, dass das Betätigungselement (64) erst verformt und danach, aufgrund der Elastizität der Membran (25), rückverformt wird.

7. Handgehaltene strombetriebene Brustpumpe nach einem vorhergehenden Anspruch, einschließlich einer Steuerschaltung (61), die dazu eingerichtet ist, Pumpentätigkeit durch Alternieren der Richtung des Motors (54) zu bewirken.

8. Handgehaltene strombetriebene Brustpumpe nach einem vorhergehenden Anspruch, wobei das Gehäuse (7) eine Pumpenkammer (122) einschließt, in der das Kolbenmittel (25) betrieben wird.

9. Batteriebetriebene handgehaltene Brustpumpe nach einem vorhergehenden Anspruch.

10. Batteriebetriebene handgehaltene Brustpumpe nach Anspruch 9, einschließlich eines Boostreglers (83) zum Regeln der Spannung, die dem Motor (54) zugeführt wird.

## Revendications

1. Tire-lait mécanique, manuel comprenant :
un corps (7) comportant une partie de réception de sein (23) et un moyen pour attacher un récipient afin de recevoir le lait exprimé ;
un moyen formant piston (25), incluant un élément d'actionnement (64), pour effectuer le pompage ; et
une unité moteur (1) incluant un moteur (54) et un mécanisme d'entraînement pour entraîner en va-et-vient le moyen formant piston (25),
dans lequel le mécanisme d'entraînement est configuré pour être relié audit élément d'actionnement quand il est amené en contact avec celui-ci,
**caractérisé en ce que** le mécanisme d'entraînement est un mécanisme à manivelle (62) et inclut un moyen formant bielle (63) et ladite liaison audit élément d'actionnement (64) est effectuée par une déviation et un rétablissement élastiques relatifs entre le moyen formant bielle (63) et ledit élément d'actionnement (64).

2. Tire-lait mécanique, manuel selon la revendication 1, dans lequel l'élément d'actionnement est une saillie pouvant être déviée élastiquement (64) se projetant depuis le moyen formant piston (25).

3. Tire-lait mécanique, manuel selon la revendication 2, dans lequel la projection (64) présente un renflement (64a) et le moyen formant bielle (63) inclut un moyen pour recevoir ledit renflement (64a) de manière à accoupler fonctionnellement le moyen formant bielle (63) et la saillie (64).

4. Tire-lait mécanique, manuel selon la revendication 3, dans lequel le moyen formant bielle (63) comporte une partie d'accouplement (63a) façonnée pour entourer la saillie (64) partiellement et le moyen pour recevoir le renflement (64a) comprend un évidement ou une fente (63b) dans ladite partie d'accouplement (63a).

5. Tire-lait mécanique, manuel selon l'une quelconque des revendications précédentes, dans lequel le moyen formant piston comprend un diaphragme élastique (25).

6. Tire-lait mécanique, manuel selon la revendication 5, dans lequel :
le mécanisme d'entraînement est un mécanisme à manivelle (62) et inclut un moyen formant bielle (63) ;
l'élément d'actionnement (64) se projette depuis le diaphragme (25) ; et
le moyen formant bielle (63) est configuré de telle sorte que ladite liaison audit élément d'actionnement (64) est effectuée par l'élément d'actionnement (64) qui d'abord dévie puis se rétablit, en raison de l'élasticité du diaphragme (25).

7. Tire-lait mécanique, manuel selon une quelconque revendication précédente, incluant un circuit de commande (61) configuré pour effectuer le pompage en alternant la direction du moteur (54).

8. Tire-lait mécanique, manuel selon une quelconque revendication précédente, dans lequel le corps (7) inclut une chambre de pompage (122) dans laquelle le moyen formant piston (25) fonctionne.

9. Tire-lait manuel alimenté par batterie selon une quelconque revendication précédente.

10. Tire-lait manuel alimenté par batterie selon la revendication 9, incluant un régulateur de suralimentation (83) pour réguler la tension apportée au moteur (54).
